# FASCICULE DE BREVET EUROPEEN

(11) **EP 4 247 310 B1**
(45) Date de publication et mention de la délivrance du brevet: **26.11.2025**
(21) Numéro de dépôt: 21793974.3
(22) Date de dépôt: 28.10.2021
(51) Int. Cl.: A61F 5/30, A61F 5/01

(54) **ORTHESE DE MAIN FLEXIBLE POUR PREVENIR OU TRAITER LE SYNDROME DU CANAL CARPIEN ET/OU DU CANAL DE GUYON**
FLEXIBLE HANDORTHESE ZUR VERMEIDUNG ODER BEHANDLUNG VON KARPALTUNNELN UND/ODER ABSPANNUNGS-KANAL-SYNDROM
FLEXIBLE HAND ORTHOSIS FOR PREVENTING OR TREATING CARPAL TUNNEL AND/OR GUYON?S CANAL SYNDROME

(30) Priorité: 20.11.2020 FR 2011920
(43) Date de publication de la demande: 27.09.2023
(73) Titulaire: Clouard, Jacques, 68720 Illfurth (FR)
(72) Inventeur: Clouard, Jacques, 68720 Illfurth (FR)
(74) Mandataire: Ipsilon
(86) Numéro de dépôt international: PCT/EP2021/080035
(87) Numéro de publication internationale: WO 2022/106177

(56) Documents cités:
- US-A- 5 810 753
- US-A1- 2003 205 232

## Description

### Domaine technique :

La présente invention concerne une orthèse de main flexible pour prévenir ou traiter le syndrome du canal carpien et/ou du canal de Guyon, comportant une pièce active sous la forme d'une plaque mince réalisée dans un matériau à haute résistance aux chocs et destinée à être placée sous la face palmaire de la main en englobant la commissure du pouce pour amortir les chocs et les vibrations, et une enveloppe disposée autour de ladite pièce active et agencée pour former un gant, un demi-gant ou une mitaine, ladite pièce active comportant à son extrémité proximale une échancrure destinée à être située au droit du canal carpien et du canal de Guyon pour créer une zone de décharge.

### Technique antérieure :

Une des causes de l'apparition du syndrome du canal carpien et/ou du canal de Guyon est due à l'effet d'appuis prolongés de la face antérieure du poignet sur une surface dure ou rigide, tel qu'un bureau, un clavier d'ordinateur, ou similaire, ainsi qu'un volant ou un guidon de motocyclette ou de bicyclette puisque la position des mains est toujours en appui plus ou moins prononcé, l'exemple ultime étant la moto sportive. D'autres causes sont dues à l'effet de vibrations répétées appliquées dans la paume de la main lors de la conduite de véhicules et/ou de l'utilisation de matériels vibrants, de flexions-extensions et torsions du poignet, et/ou de contractions constantes et prolongées de la main, etc. L'élévation de la pression au sein du canal carpien et/ou du canal de Guyon sur la face antérieure du poignet entraine une compression du nerf médian et/ou du nerf cubital par les tendons sur les os du carpe, qui induit des pertes de sensibilité et de motricité de la main accompagnées de douleurs.

L'invention s'intéresse en particulier à la pratique de la motocyclette, qui peut générer ce type de syndrome du fait de la contraction prolongée des mains sur les poignées du guidon et des vibrations engendrées par la conduite. Une étude effectuée par le demandeur a démontré que la proportion de motards victimes de ce problème s'élève à près de 30%, et que cette proportion était comparable aux études menées sur les risques professionnels quant à l'utilisation des machines vibrantes.

Différentes solutions d'orthèse de main sont proposées sur le marché pour tenter de minimiser l'apparition de ce symptôme, mais elles n'apportent pas satisfaction ou ne sont pas compatibles avec la pratique de la moto.

A titre d'exemple, il existe sur le marché du sport des mitaines rembourrées avec un produit de remplissage, tel que de la ouate, et destinées à la pratique de la bicyclette. Ces mitaines rembourrées ont une épaisseur importante qui les rend inutilisables pour la pratique de la motocyclette, car elles ne peuvent pas être enfilées dans des gants de moto, qui sont généralement très ajustés. En outre, elles ne produisent pas l'effet escompté étant donné la nature du rembourrage utilisé qui n'assure aucun amorti.

Dans certaines applications professionnelles, eu égard aux risques professionnels encourus, tels que des coupures, on utilise des gants de travail avec une inclusion de gel de silicone. Ces gants posent également le même problème que la solution précédente, dû à leur encombrement ou à leur épaisseur, qui les rend incompatibles avec des gants de moto.

Il existe également des orthèses de main adaptées pour traiter le syndrome du canal carpien et/ou du canal de Guyon qui s'étendent des doigts jusqu'au milieu de l'avant-bras mais qui sont des orthèses rigides ayant pour effet d'immobiliser le poignet, et empêchent par conséquent toute pratique de la moto. Un exemple est notamment décrit dans la publication US 5 810 753.

La publication EP 1 289 455, ou son équivalent US 2003/0205232, propose une manique permettant l'amortissement des vibrations et la prévention du syndrome du canal carpien sous la forme d'un demi-gant pourvu d'un coussin amortisseur en forme de fer à cheval délimitant un évidement central pour éviter la compression du nerf médian. Afin d'éviter que le coussin amortisseur ne s'écrase latéralement lorsqu'il est soumis à une pression et ne ferme l'évidement central, un mécanisme de maintien de l'évidement sous la forme d'un matériau rigide est disposé autour de l'évidement central. La présence de cet élément rigide dans la face palmaire de la main rend cette orthèse totalement inconfortable pour l'utilisateur. La manique peut être complétée par un système de pontage qui recouvre l'évidement central pour empêcher l'introduction d'objets externes, contribuant encore à augmenter l'inconfort de l'orthèse. En outre, elle présente une épaisseur trop importante pour être enfilée dans des gants de moto et n'englobe pas la commissure du pouce.

La publication FR 3 071 721 déposée par le demandeur propose une orthèse de main flexible, confortable et efficace, adaptée à la pratique de la moto, et facilement utilisable pour d'autres applications et d'autres domaines techniques, tels que le loisir, le sport, et le milieu professionnel. L'orthèse proposée correspond au préambule de la revendication 1 et a l'avantage d'être très mince, de l'ordre de 2 à 3 millimètres, d'épouser parfaitement la main, et de pouvoir s'enfiler aisément dans tout type de gants, notamment des gants de moto particulièrement ajustés, sans générer aucune gêne.

### Présentation de l'invention :

La présente invention propose une orthèse de main flexible perfectionnée par rapport à celle objet de la publication FR 3 071 721, qui permet de préserver les courbures physiologiques naturelles de la main, en soutenant le creux naturel dans la partie centrale de la paume de la main pour limiter voire éviter son effondrement lors d'un appui de la main sur une surface dure et/ou lors du serrage de la main autour d'une poignée, d'un guidon, d'un volant d'un véhicule, d'un engin ou d'une machine.

Dans ce but, l'invention concerne une orthèse de main du genre indiqué en préambule, caractérisée en ce qu'elle comporte en outre un dispositif anti-effondrement de la paume de la main, disposé dans une partie centrale de ladite pièce active, entre une extrémité distale et ladite extrémité proximale de ladite pièce active, et destinée à être située au droit du creux de la face palmaire de la main.

Grâce à ce perfectionnement, l'orthèse participe au maintien des courbures naturelles de la main en évitant ou en limitant l'effondrement du creux de la paume de main, ayant pour effet de diminuer les tensions dans les tendons et de détendre la main.

Ledit dispositif anti-effondrement comporte avantageusement une surépaisseur de matière formant un relief bombé, ledit relief bombé pouvant être constitué d'un coussinet en forme de dôme. Ledit coussinet peut en outre s'inscrire en vue de dessus sensiblement dans un triangle.

De manière préférentielle, ledit dispositif anti-effondrement est réalisé dans un matériau ayant des propriétés physiques différentes de celles du matériau de la pièce active. Notamment, la densité du matériau du dispositif anti-effondrement peut être inférieure à la densité du matériau de la pièce active et/ou la dureté du matériau du dispositif anti-effondrement peut être inférieure à la dureté du matériau de la pièce active

Le matériau du dispositif anti-effondrement peut être choisi dans le groupe comprenant le caoutchouc synthétique, l'éthylène-acétate de vinyle, le polyéthylène basse densité, et préférentiellement une mousse néoprène basse densité, ayant une densité comprise entre 125 et 175 kg/m³, préférentiellement égale à 150 kg/m³, et une dureté d'environ 10 Shore A.

Le matériau de la pièce active peut être choisi dans le groupe comprenant le caoutchouc synthétique, l'éthylène-acétate de vinyle, le polyéthylène basse densité, et préférentiellement une mousse néoprène haute densité, commercialisée sous la dénomination « Crispon choc plus », ayant une densité comprise entre 260 et 320 kg/m³ et de préférence égale à 290 kg/m³, et une dureté d'environ 64 à 72 Shore A.

Dans une forme préférée de l'invention, le dispositif anti-effondrement est disposé entre la pièce active et l'enveloppe. En outre, le dispositif anti-effondrement et la pièce active peuvent être assemblés par collage.

Le dispositif anti-effondrement peut avoir une épaisseur comprise entre 1mm et 3mm, et de préférence égale à 2mm dans sa partie la plus épaisse. La pièce active peut également avoir une épaisseur comprise entre 1mm et 3mm, et de préférence égale à 2mm.

### Brève description des figures :

La présente invention et ses avantages apparaîtront mieux dans la description suivante d'un mode de réalisation donné à titre d'exemple non limitatif, en référence aux dessins annexés, dans lesquels :
- La figure 1 est une vue en plan de la plaque de matière formant la partie active d'une orthèse de main selon l'invention,
- La figure 2 est une vue en perspective d'une orthèse de main selon l'invention montrant la partie active en trait plein et l'enveloppe mettant en forme ladite partie active en trait interrompu, et
- La figure 3 est une vue en coupe agrandie de l'orthèse de main de la figure 2 passant par son milieu.

### Description détaillée de l'invention :

Dans l'exemple de réalisation illustré, les éléments ou parties identiques portent les mêmes numéros de référence. En outre, les termes qui ont un sens relatif, tels que vertical, horizontal, droite, gauche, avant, arrière, au-dessus, en-dessous, proximal, distal, etc. doivent être interprétés dans des conditions normales d'utilisation de l'invention, et telles que représentées sur les figures.

En référence aux figures, l'orthèse de main 1 selon l'invention est particulièrement indiquée dans des situations où les mains sont exposées à des vibrations et dans lesquelles les vibrations sont impossibles à vaincre avec des appareillages connus d'épaisseur conséquente. Dans ce but, l'orthèse de main 1 peut présenter la forme d'une mitaine, d'un demi-gant ou d'un gant en fonction des variantes de réalisation de l'invention, et peut s'utiliser seule ou enfilée dans un autre gant, une moufle ou tout autre vêtement ou article de protection pour les mains. Elle est conçue pour absorber les chocs et les vibrations transmis aux mains lors de la pratique de sports, de loisirs, mais également dans le milieu professionnel, afin de prévenir voire de traiter le syndrome du canal carpien et/ou du canal de Guyon, qui sont des canaux ostéo-fibreux proches l'un de l'autre.

Elle comporte une partie active 2 solide, sous la forme d'une plaque mince réalisée dans un matériau à haute résistance aux chocs, et une enveloppe 3 réalisée dans un matériau souple et élastique qui entoure la partie active 2 en la maintenant et permet d'enfiler facilement l'orthèse 1 sur la main et la retirer tout aussi facilement.

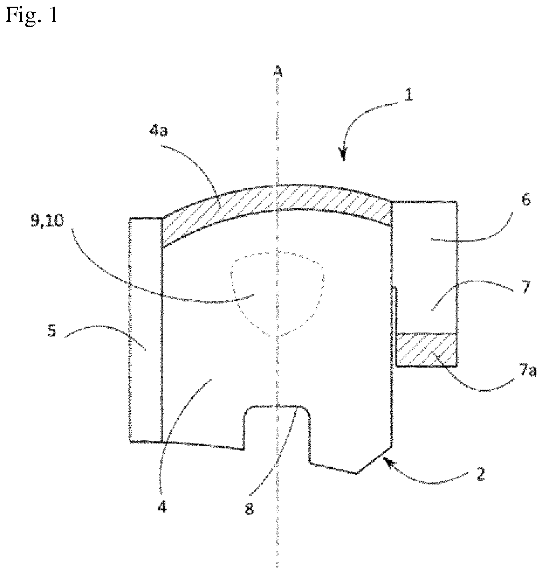

La pièce active 2 représentée à plat à la figure 1 est destinée à s'étendre sur toute la face palmaire de la main jusqu'à l'extrémité proximale des premières phalanges des doigts, et à englober le bord latéral et le bord médial de la main, ainsi que la première phalange du pouce. Elle est de préférence formée d'une seule pièce découpée dans une plaque de matière. La matière utilisée a une résistance accrue aux chocs. Elle peut être choisie dans le groupe comprenant le caoutchouc synthétique (Néoprène^{®}), l'éthylène-acétate de vinyle (EVA), le polyéthylène basse densité (LDPE), et similaires. On privilégiera une plaque en mousse néoprène haute densité, de faible épaisseur entre 1 et 3 mm et de préférence égale à 2 mm, telle que celle commercialisée sous la dénomination « Crispon choc plus » et utilisée essentiellement en podologie pour les semelles orthopédiques. Il s'agit dans ce cas d'un néoprène dont la densité peut être comprise en 260 et 320 kg/m³ et de préférence égale à 290 kg/m³ (selon la norme DIN EN ISO 845). Ce néoprène peut en outre présenter une dureté comprise en 64 et 72 Shore A. Bien entendu, cet exemple de matière et ces valeurs ne sont pas limitatifs et tout autre matériau équivalent avec des valeurs équivalentes peut convenir. L'avantage de ce choix de matériau préféré réside dans sa très haute résistance aux chocs, qui se traduit par une résistance accrue à l'écrasement, une excellente résilience et une absorption importante des vibrations, au regard de sa très faible épaisseur, ce qui n'est le cas d'aucune autre classe de matériaux testée à ce jour. Par ailleurs, ce matériau est en outre recouvert d'un tissu de type jersey comprenant des fils d'argent dont les vertus antiseptiques sont primordiales pour un usage dans un environnement confiné tel que peut l'être celui d'un gant. Ainsi, ce choix de matériau combine des fonctionnalités tout à fait avantageuses pour obtenir une orthèse de main 1 optimale.

En référence à la figure 1, la pièce active 2 comporte une partie centrale 4 qui correspond en forme et en dimension sensiblement à la face palmaire de la main, un bord latéral 5 droit et un bord médial 6 gauche prolongé par une partie médiale 7 libre qui correspond en forme et en dimension sensiblement à la première phalange du pouce qui forme la commissure du pouce. La pièce active 2 forme une couche de matière intermédiaire entre la main et la poignée, le guidon, le volant ou la surface dure sur laquelle repose la main, ayant pour effet d'absorber les vibrations et les pressions, permettant de diminuer voire de supprimer la transmission de ces ondes de chocs dans les tendons et de détendre la main. En outre, en englobant la commissure du pouce, la pièce active 2 a pour effet d'augmenter le diamètre de fermeture de la main, permettant de diminuer la tension dans les tendons et de participer à la détente de la main.

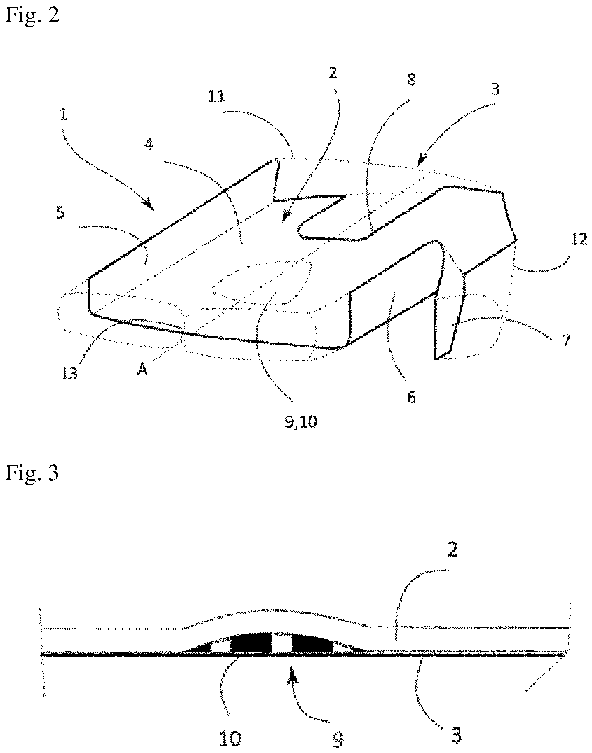

La figure 2 permet de mieux appréhender la pièce active 2 mise en forme par l'enveloppe 3. Les extrémités distales de la partie centrale 4 et de la partie médiale 7 de la pièce active sont amincies par un bord coupé en biseau 4a, 7a, représenté par des hachures. Ce bord aminci permet d'éviter toute rupture brutale d'épaisseur qui pourrait se révéler être inconfortable pour l'utilisateur.

La zone proximale de la partie centrale 4 de la pièce active 2 comporte une échancrure 8, formée par une découpe dans la plaque de matière. L'échancrure 8 peut présenter une forme en U ou similaire, centrée sur l'axe médian A de l'orthèse 1. Cette échancrure 8 est destinée à être située au droit du nerf médian et du nerf cubital respectivement dans le canal carpien et le canal de Guyon de la main, entre les reliefs de la base de la paume de main appelés éminence Thénar et éminence hypothénar. Ainsi, la pièce active 2 prend appui sur toute la face palmaire de la main, y compris sur les reliefs de la base de la paume de main, à l'exception de la zone du canal carpien et/ou du canal de Guyon. Cette échancrure 8 crée une zone de décharge au niveau du canal carpien et/ou du canal de Guyon, et évite le contact avec l'orthèse 1 et la poignée, le guidon, le volant ou la surface dure sur laquelle repose la main. Elle empêche ainsi la compression des nerfs responsables desdits syndromes et améliore de manière significative l'effet protecteur de l'orthèse 1.

En référence également à la figure 3, la pièce active 2 comporte en outre un dispositif anti-effondrement 9 de la paume de la main. Ce dispositif anti-effondrement 9 est disposé dans la partie centrale 4 de ladite pièce active 2 et est destinée à être située au droit du creux de la face palmaire de la main. Il comporte une surépaisseur de matière formant un relief bombé. Ce relief bombé est réalisé par un coussinet 10 en forme de dôme. Ce coussinet 10 peut s'inscrire en vue de dessus (figure 1) sensiblement dans un triangle ou similaire. Il est centré sur l'axe médian A de l'orthèse 1 et disposé sensiblement entre l'extrémité distale et l'extrémité proximale de la partie centrale 4. La pointe du triangle est dirigée vers l'échancrure 8. Les dimensions et le positionnement du coussinet 10 sont variables et adaptées à la morphologie de la main. Dans tous les cas, le coussinet 10 a une largeur qui correspond sensiblement à la largeur des 3e et 4e doigts, à savoir le majeur et l'annulaire ; il est situé en arrière des têtes des métacarpes d'environ 1,5 à 2 cm et à distance de l'échancrure d'environ 2 cm, étant précisé que ces valeurs ne sont pas limitatives, et sont déterminées par la taille de la main donc de l'orthèse.

Le dispositif anti-effondrement 9 est réalisé dans un matériau ayant des propriétés physiques différentes de celles du matériau de la pièce active, notamment une densité inférieure à la densité du matériau de la pièce active, et une dureté inférieure à la dureté du matériau de la pièce active. A titre d'exemple, le dispositif anti-effondrement 9 peut être réalisé dans un matériau ayant une densité comprise entre 125 et 175 kg/m³, et de préférence égale à 150 kg/m³ (selon la norme DIN EN ISO 845). Il peut en outre avoir une dureté d'environ 10 Shore A.

Le matériau du dispositif anti-effondrement 9 est donc différent de celui de la pièce active, et peut être choisi dans le groupe comprenant le caoutchouc synthétique (Néoprène^{®}), l'éthylène-acétate de vinyle (EVA), le polyéthylène basse densité (LDPE), et similaires. On privilégiera un coussinet 10 en mousse néoprène basse densité, de faible épaisseur entre 1 et 3mm et de préférence 2mm dans la partie la plus épaisse.

Le dispositif anti-effondrement 9 est avantageusement disposé entre la pièce active 2 et l'enveloppe 3, comme représenté à la figure 3. Il peut être assemblé à la pièce active 2 par collage au moyen d'une colle, d'un film adhésif intégré à la plaque active 2, un ruban adhésif double-face intercalé entre les deux, ou tout autre moyen d'assemblage adéquat. Il permet à la main de conserver ses courbures physiologiques naturelles et au creux de la paume de main de ne pas trop s'affaisser sous l'effet d'une pression importante par appui sur une poignée, un guidon, un volant, ou toute surface dure. En combinaison avec la pièce active 2, il contribue à éviter la compression des nerfs responsables des syndromes du canal carpien et/ou du canal de Guyon, et améliore encore d'avantage l'effet protecteur de l'orthèse 1. En effet, l'utilisation d'un matériau basse densité et moins dur a l'avantage de s'écraser légèrement et d'éviter d'augmenter les pressions au niveau de la paume de la main qui se produisent inévitablement lors de certaines phases de mouvement de la main sur une surface d'appui quelle qu'elle soit.

L'enveloppe 3 de l'orthèse à main 1 selon l'invention est réalisée dans un tissu extensible et respirant par exemple sous la forme d'un tissu en maille. L'enveloppe 3 peut être formée d'une seule pièce tubulaire sans couture, d'une seule pièce à plat pliée et assemblée, ou de plusieurs pièces assemblées. L'assemblage peut être effectuée par couture. Dans ce cas, la ou les pièces de tissu sont découpées de sorte que les coutures soient positionnées sous la partie active 2, afin qu'à aucun moment elles ne gênent le confort de la main de l'utilisateur. Le tissu comporte avantageusement de l'élasthanne, tel que le Lycra^{®}. L'enveloppe 3 est souple et élastique pour pouvoir être aisément enfilée sur la main et retirée de la main. Elle est également lisse et sans relief pour pouvoir être aisément enfilée dans un gant et retirée dudit gant. L'enveloppe 3 représentée en trait interrompu à la figure 2 forme une mitaine sans que cet exemple ne soit limitatif. La mitaine comporte un tube principal 11 pour épouser les faces palmaire et dorsale de la main jusqu'aux extrémités proximales des premières phalanges des doigts, et un tube secondaire 12 sur le côté droit ou le côté gauche pour épouser le pouce de la main. L'enveloppe 3 affleure l'extrémité proximale de la partie active 2 en direction du poignet et déborde de l'extrémité distale de la partie active 2 en direction des extrémités distales des premières phalanges des doigts.

Un point de couture 13 peut être prévu par exemple entre le 3e et 4e doigts pour retenir la partie active 2 à l'intérieur de l'enveloppe 3 et l'empêcher de glisser lors de l'enfilage et du retrait de l'orthèse 1. L'enveloppe 3 peut en outre être assemblée à la partie active 2 de l'orthèse 1 par collage ou tout moyen d'assemblage adéquat. On peut utiliser des points de couture mais on veillera à les disposer en dehors de la partie active 2 pour ne pas gêner le confort de l'utilisateur, et ne pas amoindrir ou pénaliser l'efficacité de l'orthèse de main 1.

La présente invention n'est bien entendu pas limitée à l'exemple de réalisation décrit mais s'étend à toute modification et variante évidentes pour un homme du métier dans la limite des revendications annexées.

## Revendications

1. Orthèse de main (1) pour prévenir ou traiter le syndrome du canal carpien et/ou du canal de Guyon, comportant une pièce active (2) sous la forme d'une plaque mince réalisée dans un matériau à haute résistance aux chocs et destinée à être placée sous la face palmaire de la main en englobant la commissure du pouce pour amortir les chocs et les vibrations, et une enveloppe (3) disposée autour de ladite pièce active (2) et agencée pour former un gant, un demi-gant ou une mitaine, ladite pièce active (2) comportant à son extrémité proximale une échancrure (8) destinée à être située au droit du canal carpien et du canal de Guyon pour créer une zone de décharge, **caractérisée en ce que** ladite orthèse à main (1) comporte en outre un dispositif anti-effondrement (9) de la paume de la main, disposé dans une partie centrale (4) de ladite pièce active (2), entre une extrémité distale et ladite extrémité proximale de ladite pièce active (2), et destinée à être située au droit du creux de la face palmaire de la main.

2. Orthèse de main selon la revendication 1, **caractérisée en ce que** ledit dispositif anti-effondrement (9) comporte une surépaisseur de matière formant un relief bombé.

3. Orthèse de main selon l'une quelconque des revendications précédentes, **caractérisée en ce que** le relief bombé du dispositif anti-effondrement (9) est constitué d'un coussinet (10) en forme de dôme qui s'inscrit en vue de dessus sensiblement dans un triangle.

4. Orthèse de main selon l'une quelconque des revendications précédentes, **caractérisée en ce que** ledit dispositif anti-effondrement (9) est réalisé dans un matériau ayant des propriétés physiques différentes de celles du matériau de la pièce active (2).

5. Orthèse de main selon la revendication 4, **caractérisée en ce que** la densité du matériau du dispositif anti-effondrement (9) est inférieure à la densité du matériau de la pièce active (2) et/ou la dureté du matériau du dispositif anti-effondrement (9) est inférieure à la dureté du matériau de la pièce active (2).

6. Orthèse à main selon l'une quelconque des revendications précédentes, **caractérisée en ce que** le matériau du dispositif anti-effondrement (9) est choisi dans le groupe comprenant le caoutchouc synthétique, l'éthylène-acétate de vinyle, le polyéthylène basse densité, et une mousse néoprène ^{®} basse densité, ayant une densité comprise entre 125 et 175 kg/m³, selon la norme DIN EN ISO 845, préférentiellement égale à 150 kg/m³, et une dureté d'environ 10 Shore A.

7. Orthèse de main selon l'une quelconque des revendications précédentes, **caractérisée en ce que** le matériau de la pièce active (2) est choisi dans le groupe comprenant le caoutchouc synthétique, l'éthylène-acétate de vinyle, le polyéthylène basse densité, et préférentiellement une mousse néoprène ^{®} haute densité, ayant une densité comprise entre 260 et 320 kg/m³, selon la norme DIN EN ISO 845, et de préférence égale à 290 kg/m³, et une dureté d'environ 64 à 72 Shore A.

8. Orthèse de main selon l'une quelconque des revendications précédentes, **caractérisé en ce que** le dispositif anti-effondrement (9) est disposé entre la pièce active (2) et l'enveloppe (3).

9. Orthèse de main selon l'une quelconque des revendications précédentes, **caractérisée en ce que** le dispositif anti-effondrement (9) et la pièce active (2) sont assemblés par collage.

10. Orthèse de main selon l'une quelconque des revendications précédentes, **caractérisée en ce que** le dispositif anti-effondrement (9) a une épaisseur comprise entre 1mm et 3mm, et de préférence égale à 2mm dans sa partie la plus épaisse, et **en ce que** la pièce active (2) a une épaisseur comprise entre 1mm et 3mm, et de préférence égale à 2mm.

## Patentansprüche

1. Handorthese (1) zur Vorbeugung oder Behandlung des Karpaltunnelsyndroms und/oder des Guyon-Kanal-Syndroms, mit einem aktiven Teil (2) in Form einer dünnen Platte aus einem hochschlagfesten Material, das unter die Handfläche der Hand gelegt wird, wobei es den Daumenballen umschließt, um Stöße und Vibrationen zu dämpfen, und eine Hülle (3), die um das aktive Teil (2) herum angeordnet und so ausgebildet ist, dass sie einen Handschuh, einen Halbhandschuh oder einen Fingerhandschuh bildet, wobei das aktive Teil (2) an seinem proximalen Ende eine Aussparung (8) aufweist, die dazu bestimmt ist, sich in Höhe des Karpaltunnels und des Guyon-Kanals zu befinden, um einen Entlastungsbereich zu schaffen, **dadurch gekennzeichnet, dass** die Handorthese (1) außerdem eine Vorrichtung (9) gegen das Zusammenfallen der Handfläche umfasst, die in einem mittleren Teil (4) des aktiven Teils (2) zwischen einem distalen Ende und dem proximalen Ende des aktiven Teils (2) angeordnet ist und dazu bestimmt ist, sich in Höhe der Handflächenmulde zu befinden.

2. Handorthese nach Anspruch 1, **dadurch gekennzeichnet, dass** die Einsturzschutzvorrichtung (9) eine Materialverdickung aufweist, die ein gewölbtes Relief bildet.

3. Handorthese nach einem der vorstehenden Ansprüche, **dadurch gekennzeichnet, dass** die gewölbte Erhebung der Kollabierungsschutzvorrichtung (9) aus einem kuppelförmigen Polster (10) besteht, das in der Draufsicht im Wesentlichen in einem Dreieck liegt.

4. Handorthese nach einem der vorstehenden Ansprüche, **dadurch gekennzeichnet, dass** die Einsturzschutzvorrichtung (9) aus einem Material hergestellt ist, das andere physikalische Eigenschaften als das Material des aktiven Teils (2) aufweist.

5. Handorthese nach Anspruch 4, **dadurch gekennzeichnet, dass** die Dichte des Materials der Kollapsvorrichtung (9) geringer ist als die Dichte des Materials des aktiven Teils (2) und/oder die Härte des Materials der Kollapsvorrichtung (9) geringer ist als die Härte des Materials des aktiven Teils (2).

6. Handorthese nach einem der vorstehenden Ansprüche, **dadurch gekennzeichnet, dass** das Material der Kollapsvorrichtung (9) aus der Gruppe ausgewählt ist, die synthetischen Kautschuk, Ethylen-Vinylacetat, Polyethylen niedriger Dichte und einen Neopren^{®}-Schaum niedriger Dichte umfasst, mit einer Dichte zwischen 125 und 175 kg/m3 gemäß DIN EN ISO 845, vorzugsweise 150 kg/m 3 , und einer Härte von etwa 10 Shore A.

7. Handorthese nach einem der vorstehenden Ansprüche, **dadurch gekennzeichnet, dass** das Material des aktiven Teils (2) aus der Gruppe ausgewählt ist, die synthetischen Kautschuk, Ethylen-Vinylacetat, Polyethylen niedriger Dichte und vorzugsweise einen Neopren^{®}-Schaumstoff hoher Dichte umfasst, mit einer Dichte zwischen 260 und 320 kg/m3 und vorzugsweise 290 kg/m3 und einer Härte von etwa 64 bis 72 Shore A.

8. Handorthese nach einem der vorstehenden Ansprüche, **dadurch gekennzeichnet, dass** die Kollapsvorrichtung (9) zwischen dem aktiven Teil (2) und der Hülle (3) angeordnet ist.

9. Handorthese nach einem der vorstehenden Ansprüche, **dadurch gekennzeichnet, dass** die Kollapsvorrichtung (9) und das aktive Teil (2) durch Kleben miteinander verbunden sind.

10. Handorthese nach einem der vorstehenden Ansprüche, **dadurch gekennzeichnet, dass** die Kollapsvorrichtung (9) eine Dicke zwischen 1 mm und 3 mm, vorzugsweise 2 mm an ihrer dicksten Stelle, aufweist und dass das aktive Teil (2) eine Dicke zwischen 1 mm und 3 mm, vorzugsweise 2 mm, aufweist.

## Claims

1. A hand orthosis (1) for preventing or treating carpal tunnel syndrome and/or Guyon's canal syndrome, comprising an active part (2) in the form of a thin sheet produced from a material with a high impact strength and intended to be placed under the palmar face of the hand and embracing the commissure of the thumb in order to dampen shocks and vibrations, and an envelope (3) disposed around said active part (2) and arranged to form a glove, a halfglove or a mitt, said active part (2) comprising an indentation (8) at its proximal end intended to be positioned level with the carpal tunnel and the Guyon's canal in order to generate a load relief zone, **characterized in that** said hand orthosis(1) further comprises an anti-collapse device (9) which prevents the collapse of the palm of the hand, disposed in a central portion (4) of said active part (2) between a distal end and said proximal end of said active part (2), and intended to be located level with the hollow of thepalmar face of the hand.

2. The hand orthosis according to claim 1, **characterized in that** said anti-collapse device (9) comprises a thickened material forming a domed topography.

3. The hand orthosis according to claim 2, **characterized in that** the domed topography of the anti-collapse device (9) is constituted by a pad with a domed shape which is substantially inscribed inside a triangle in plan view.

4. The hand orthosis according to any one of the preceding claims, **characterized in that** said anti-collapse device (9) is produced from a material having physical properties which differ from those of the material of the active part(2).

5. The hand orthosis according to claim 4, **characterized in that** the density of the material of the anti-collapse device (9) is lower than the density of the material of the active part (2) and/or the hardness of the material of the anti-collapse device (9) is less than the hardness of the material of the active part(2).

6. The hand orthosis according to any one of the preceding claims, **characterized in that** the material of the anti-collapse device (9) is selected from the group comprising synthetic rubber, ethylene-vinyl acetate, low density polyethylene, and preferably a low density neoprene foam with a density in the range125 to 175 kg/m3, preferably equal to 150 kg/m3, and a hardness of approximately 10 Shore A.

7. The hand orthosis according to any one of the preceding claims, **characterized in that** the material of the active part (2) is selected from the group comprising synthetic rubber, ethylene-vinyl acetate, low density polyethylene, and preferably a high density neoprene foam, marketed under the trade name "Crispon choc plus", with a density in the range 260 to 320 kg/m3 and preferably equal to 290 kg/m3, and a hardness of approximately 64 to 72 Shore A.

8. The hand orthosis according to any one of the preceding claims, **characterized in that** the anti-collapse device (9) is disposed between the active part (2) and the envelope (3).

9. The hand orthosis according to any one of the preceding claims, **characterized in that** the anti-collapse device (9) and the active part (2) are assembled by bonding.

10. The hand orthosis according to any one of the preceding claims, **characterized in that** the anti-collapse device (9) has a thickness in the range 1 mm to 3 mm, and preferably equal to 2 mm at its thickest part, and **in that** the active part (2) has a thickness in the range 1 mm to 3 mm, and preferably equal to 2 mm.
